(19) **Europäisches Patentamt**

**European Patent Office**

**Office européen des brevets**

(11) **EP 0 627 617 B1**

(12) # EUROPEAN PATENT SPECIFICATION

(45) Date of publication and mention
of the grant of the patent:
**04.02.1998  Bulletin 1998/06**

(51) Int Cl.6: **G01M 11/08**, G01N 3/08

(21) Application number: **94850080.6**

(22) Date of filing: **13.05.1994**

(54) **Method and device for the mechanical testing of optical fibre**

Verfahren und Vorrichtung für die mechanische Prüfung von optischen Fasern

Procédé et dispositif d'essai mécanique d'un fibre optique

(84) Designated Contracting States:
**CH DE ES FR GB IT LI NL**

(72) Inventor: **Svensson, Torbjörn
S-134 37 Gustavsberg (SE)**

(43) Date of publication of application:
**07.12.1994  Bulletin 1994/49**

(56) References cited:
**EP-A- 0 449 491          US-A- 4 940 891**

(73) Proprietor: **TELIA AB
123 86 Farsta (SE)**

- **REVIEW OF SCIENTIFIC INSTRUMENTS, vol.64, no.1, January 1993, New York US, pp. 250-252; R.E. MEDRANO et al.: 'Optical System to Detect Fracture of Glass Materials'**

## Description

### TECHNICAL FIELD

The present invention relates to a method and a device according to the preamble of claims 1 and 3, respectively.

The optic fibre is made of glass or some other material allowing the transfer of light. The fibres are intended for use in telecommunications and data-transmission. The testing relates to determination of the susceptibility of a fibre to mechanical fatigue, which governs the working life of the fibre.

The invention is intended for use in conjunction with type testing of optic fibre, research objectives, in studies in different environments etc.

### PRIOR ART

In testing the ageing characteristics of fibres, it is previously known to arrange the fibres in different forms of tensile test. A number of fibres with chosen loads are arranged in a test pattern. The fibre is thereafter allowed to remain in the load position until fracture of the fibre occurs. In order to obtain a statistical selection, a large number of tests are arranged, at the same load, in a measuring apparatus. The arrangement is realized, for example, in a tensile testing machine or the like. The equipment which is used in these tests is relatively expensive and/or space-hungry and remains occupied whilst the measurement series is conducted. In conducting a measurement series, a large number of test set-ups have to be arranged.

US patent 4 940 891 discloses a system that automatically measures the strength of optical fibres by initiating a series of breaks in an optical fibre delivered from a supply spool. The fibre is stressed to failure in an automatically displacable jaw mechanism and the fibre is advanced and the measurement repeated. A computer-controller advances the fibre, records data and monitors jaw mechanism fracture parameters so that the strength distribution of a length of optical fibre is reliably determined.

Patent EP 361962 and SU 1357713 show devices in the form of a cone having spiral-shaped recesses. The fibre is exposed to a gradually increased deformation, whereupon detection takes place of the escaped light.

### ACCOUNT OF THE INVENTION

### TECHNICAL PROBLEM

There is a need to calculate, in a simple manner, the working life of a fibre. In order to do this, it is desirable to present a large number of measurement results based on a small number of tests. There is therefore a need to create a large number of fractures of the fibre in a single test. It shall herein be possible to determine the relation of the fracture to the tensile load to which the fibre is exposed. It is further desirable to attain significant results, in the static testing of fibres, from separate test patterns.

It is further desirable that testing should be able to be carried out on a large number of different specimens at the same time and that these should constitute, amongst themselves, different fibre types. To expose a fibre to different loads under a single test and to obtain measurement results which are relevant is also desirable.

The object of the present invention is to solve the defined problem.

### THE SOLUTION

The above-mentioned object is achieved by means of a method and a device according to the characterizing portions of claims 1 and 3, respectively. The fibre, which is used for light-based communications transmission, is bent in a number of turns. Each turn has a predetermined diameter or is arranged within certain maximum and minimum diameters. The choice of diameter is dependent upon the diameter of the fibre, strength characteristics etc. The turns which have been arranged on the fibre are fixed. The fibre which has thus been fixed is kept in this position for a period of time. During the said time period, the fibre is exposed to a static load. Using an expedient choice of maximum and minimum diameters for the turns, the test can be controlled to suitable test times which are able to be predicted.

During the said time period, fractures of the fibre appear. The said fractures occur at different times. The time when the fracture occurred is registered for each fracture. The test is conducted for a predetermined time period or until a predetermined number of fractures has been registered.

A measurement series is obtained providing information on when a fracture has occurred. The obtained measurement series is used to determine the fatigue characteristics of the fibre. For example, the stress corrosion coefficient, n, can be determined from

$$n = N / (\ln(R) \; \Delta k / \Delta \ln(ts)),$$

where

N indicates the number of turns in which the fibre is arranged,
R denotes the relationship between the radius of the maximum and minimum turns,
$\Delta k / \Delta \ln(ts)$ relates to the rate of change of measurement data,
k denotes the serial number for a particular fracture and ts the time of occurrence of the corresponding fracture.

The invention further relates to a device for the realization of the method. The device comprises an element on which the fibre is wound in a number of turns, the turns being circular or at least partly circular. In order to achieve circular or partly circular turns, the element is cylindrical or provided with a surface which is partly cylindrical. The cylindrical surface is also allowed to be conical, having predetermined maximum and minimum diameters. The diameters are determined with regard to the diameter and strength characteristics of the fibre. When the fibre is fitted to the element, the fibre is fixed in this position for a period of time.

The element around which the fibre has been wound in a number of turns is thereafter released or can remain in place until the measurement is conducted. In the event of the windings having been fitted around a deformable cylindrical element or the element having been removed after the fibre has been wound around the element, it is possible, by mechanical action, to cause the turns in the fibre to assume the said turns between a maximum and a minimum diameter.

A detecting element registers the occurrence of fractures on the fibre and the time of the event in question. The information is transferred to a computing element which, on the basis of acquired data, calculates the ageing characteristics of the fibre.

ADVANTAGES

The present invention has the advantage that expensive equipment is not occupied throughout the testing period. In addition, devices for exposing the fibre to tensile load do not need to be used.

To wind the fibre around a device is simple and requires no special forces. A single fibre is allowed, moreover, to acquire a plurality of fractures, whereas previous methods only permitted stress until a fracture occurred in the fibre.

The fact that the invention is simple to use produces the low costs of each series of tests.

DESCRIPTION OF FIGURES

Fig. 1     shows a fibre arranged in a number of turns.
Fig. 2     shows an element in conical construction.
Fig. 3     shows an element having a partly circular surface.
Fig. 4     shows an element in cylindrical construction.
Fig. 5     shows the stress and contraction forces in a bent fibre.

PREFERRED EMBODIMENT

The inventive concept is described below with reference to the figures and notations therein.

The invention relates to an optic fibre which is exposed to static load for a period of time. In order to achieve the load, the fibre is wound in a number of turns.

Each turn is herein given a diameter which is determined by the diameter and strength characteristics of the fibre. A fibre of small diameter is bent more than a fibre of large diameter.

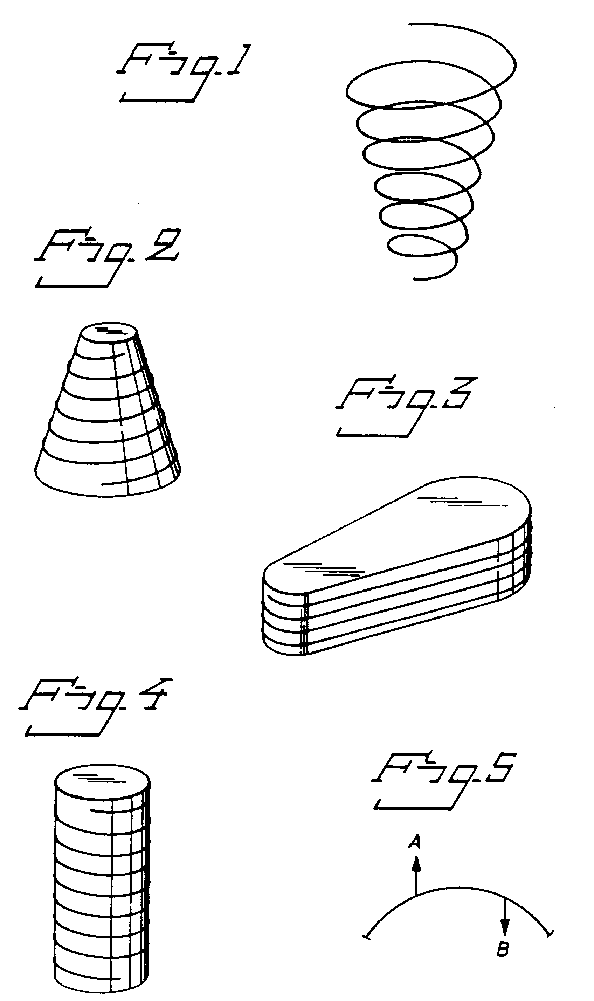

The desired bending is effected by means of an element which gives the fibre a defined bending diameter. The diameter is defined to a maximum and minimum value. In a first embodiment, the fibre is wound around a conical element according to Fig. 2. A second embodiment is shown in Fig. 3, in which the fibre is wound around an element having a partly circular surface. In addition, an embodiment is exemplified in Fig. 4 in which the fibre is wound arounda circular surface. In order to achieve different diameters of the different turns, in the latter case the fibre is released from the element. Following its release, the fibre is exposed to a mechanical action which causes the turns of the fibre to assume different diameters, which are defined between a maximum and a minimum value. The fibre is thereafter arranged in a number of turns according to Fig. 1. In all illustrative embodiments, the fibre is fixed such that a fracture of one turn cannot affect other turns.

In a variant of the embodiment having a cylindrical element, this is constructed in a deformable material. In this case, the fibre is allowed to remain on the element. The fibre and the element are thereafter subjected to mechanical action, whereupon the desired maximum and minimum diameter of the turns of the fibre are obtained. In this case, material which recovers its original shape can be used if the mechanical action is allowed to operate throughout the duration of the test. It is also possible to use deformable material which does not recover its original shape. In this case, the element is subjected to mechanical action for the time required for the said element to assume its desired shape.

By virtue of the fact that the fibre is arranged in a number of turns, the diameter of which is related to the diameter of the fibre, the fibre comes to be exposed to a static load. The fibre is exposed partly to a tensile stress, A, partly to a contraction force, B. During the duration of the test, fractures materialize on the fibre. These fractures appear at different times. During the testing, a certain number of fractures are required to occur on the fibre. Based on the known characteristics and dimensions of the fibre, an estimate is made of the time for which the fibre is to undergo the test. Alternatively, a pre-set number of fractures has to have occurred on the fibre before the test is terminated. The invention therefore allows the fibre to be exposed to forces which are known, without any weights, tension devices, etc. being used.

During the time in which the fibre undergoes testing, a detecting element registers when fractures occur on the fibre. The information is transferred to a computing element, which compiles the information. The computing element calculates, on the basis of received information, the tension corrosion coefficient, n, for example, of the fibre. n is obtained from

$$n = N/ (\ln(R)\ \Delta k/\Delta \ln(ts)),$$

where

N denotes the number of turns,
R denotes the relationship between the radius of the maximum and minimum turn,
$\Delta k/\Delta \ln(ts)$ denotes the declination of measurement data in a linear adjustment, where k denotes the serial number for a fracture and ts the time of the corresponding fracture.

By virtue of n having been established, the ageing characteristics of the fibre are determined. The working life of the fibre is determined therefrom using methods which are known per se and are known to experts within the fibre engineering field.

The invention is not limited to the embodiment shown above, but can be subject to modifications within the scope of subsequent patent claims 1-7.

**Claims**

1. Method for determining the ageing characteristics of a fibre, wherein the fibre is bent in a number of turns, the diameters of which range between a maximum and a minimum value, and is fixed in this state, **characterized** in that the occurrence of a series of fractures in the fibre, resulting from static load, is registered in a detecting element, as well as the time of occurence of the fractures, which registered information in said detecting element is transferred to a computing element, which calculates, on the basis of said information, the stress corrosion coefficient, n, of the fibre using the following formula:

$$n = N/ (\ln(R)\ \Delta k/\Delta \ln(ts)),$$

where

N denotes the number of turns,
R denotes the relationship between the radius of the maximum and minimum turn,
$\Delta k/\Delta \ln(ts)$ denotes the rate of change of measurement data, where k denotes the serial number for a fracture and ts the time of occurrance of the corresponding fracture, wherein the stress corrosion coefficient, n, defines the ageing characteristics of the fibre.

2. Method according to claim 1, **characterized** in that the diameter of the turns is determined by the diameter and strength characteristics of the fibre.

3. Device, comprising an element having at least one partly circular surface, the diameter of which is determined by the diameter and strength characteristics of a fibre, for determination of the working life of the fibre, **characterized** in that the fibre is fitted to the element, over the at least partly circular surface, in a number of turns for a period of time during which a detecting element registers the occurrence of a series of fractures in the fibre and the time of occurrence of each fracture, the information on the number of break-offs and the time of the break-offs being transferred from said detecting element to a computing element which calculates the stress corrosion coefficient, n, of the fibre, using the following formula:

$$n = N/ (\ln(R)\ \Delta k/\Delta \ln(ts)),$$

where

N denotes the number of turns,
R denotes the relationship between the radius of the maximum and minimum turn,
$\Delta k/\Delta \ln(ts)$ denotes the rate of change of measurement data, where k denotes the serial number for a fracture and ts the time of occurrance of the corresponding fracture, wherein the stress corrosion coefficient, n, defines the ageing characteristics of the fibre, and that the maximum and minimum diameter of the partly circular surface is determined with regard to the diameter and known strength characteristics of the tested fibre.

4. Device according to claim 3, **characterized** in that the element is conical.

5. Device according to claim 3, **characterized** in that the fibre is fitted to the element in one or more turns, the detection of fibre fractures being realized, for example, acoustically, mechanically or optically using a detecting element.

6. Device according to claim 3, **characterized** in that the element is cylindrical and the fibre, after having been wound around the element, is either released or, if the cylinder is made from deformable material, remains seated on the cylinder, whereafter the different turns of the fibre, by means of mechanical action, are caused to assume different diameters, which are defined to a maximum and a minimum value.

7. Device according to claims 3-6, **characterized** in that the bending diameter of the fibre around the circular surface indicates the load to which the fibre is exposed.

**Patentansprüche**

1. Verfahren zum Bestimmen der Alterungscharakteristiken einer Faser, wobei die Faser in einer Anzahl von Windungen gekrümmt wird, deren Durchmesser zwischen einem maximalen und einem minimalen Wert liegen und in diesem Zustand fixiert wird, dadurch gekennzeichnet, daß das Auftreten einer Reihe von Brüchen in der Faser, die von statischer Belastung resultieren, in einem Detektionselement und auch die Zeit des Auftretens der Brüche registriert wird, welche registrierte Information in dem Detektionselement zu einem Rechnerelement übertragen wird, das aufgrund dieser Information den Spannungskorrosionskoeffizienten n der Faser unter Verwendung der folgenden Formel berechnet:

$$n = N/ (\ln(R) \; \Delta k/\Delta \ln(ts))$$

wobei

N die Anzahl von Windungen bezeichnet,
R die Beziehung zwischen dem Radius der maximalen und minimale Windung bezeichnet
$\Delta k/\Delta \ln(ts)$ die Änderungsrate der Meßdaten bezeichnet, wobei
k die Seriennummer für einen Bruch und
ts die Zeit des Auftretens des entsprechenden Bruchs bezeichnet, wobei der Spannungskorrosionskoeffizient n die Alterungscharakteristiken der Faser definiert.

2. Verfahren nach Anspruch 1, dadurch gekennzeichnet, daß der Durchmesser der Windungen durch den Durchmesser und die Stärkecharakteristiken der Faser bestimmt wird.

3. Vorrichtung, die ein Element aufweist, das wenigstens eine teilweise kreisförmige Oberfläche aufweist, deren Durchmesser durch den Durchmesser und die Stärkecharakteristiken einer Faser bestimmt ist, zum Bestimmen der Betriebs lebensdauer der Faser, dadurch gekennzeichnet, daß die Faser am Element über die wenigstens teilweise kreisförmige Oberfläche in einer Anzahl von Windungen während einer gewissen Zeitdauer angebracht wird, während der ein Detektionselement das Auftreten einer Reihe von Brüchen in der Faser und die Zeit des Auftretens jedes Bruchs registriert, wobei die Information über die Anzahl von Brüchen und die Zeit der Brüche von dem Detektionselement zu einem Rechnerelement übertragen wird, das den Spannungskorrosionskoeffizienten n der Faser unter Verwendung der folgenden Formel berechnet:

$$n = N/ (\ln(R) \; \Delta k/\Delta \ln(ts))$$

worin

N die Anzahl von Windungen bezeichnet,
R die Beziehung zwischen dem Radius der maximalen und minimalen Windung bezeichnet,
$\Delta k/\Delta \ln(ts)$ die Änderungsrate der Meßdaten bezeichnet, wobei
k die Seriennummer für einen Bruch und
ts die Zeit des Auftretens des entsprechenden Bruches bezeichnet, wobei der Spannungskorrosionskoeffizient n die Alterungscharakteristiken der Faser definiert, und daß der maximale und der minimale Durchmesser der teilweise kreisförmigen Oberfläche im Hinblick auf den Durchmesser und die bekannten Stärkeeigenschaften der untersuchten Faser bestimmt wird.

4. Vorrichtung nach Anspruch 3, dadurch gekennzeichnet, daß das Element konisch ist.

5. Vorrichtung nach Anspruch 3, dadurch gekennzeichnet, daß die Faser an einem Element in einer oder mehreren Windungen angebracht wird, wobei die Detektion von Faserbrüchen z. B. akustisch, mechanisch oder optisch unter Verwendung eines Detektionselements realisiert wird.

6. Vorrichtung nach Anspruch 3, dadurch gekennzeichnet, daß das Element zylindrisch ist und die Faser, nachdem sie um das Element herumgewikkelt worden ist, entweder gelöst wird oder, falls der Zylinder aus verformbarem Material hergestellt ist, auf dem Zylinder sitzen bleibt, woraufhin anschließend die unterschiedlichen Windungen der Faser mit Hilfe einer mechanischen Wirkung dazu gebracht werden, unterschiedliche Durchmesser anzunehmen, die bis zu einem maximalen und einem minimalen Wert begrenzt sind.

7. Vorrichtung nach den Ansprüchen 3 bis 6, dadurch gekennzeichnet, daß der Krümmungsdurchmesser der Faser um die kreisförmige Oberfläche die Last anzeigt, der die Faser ausgesetzt ist.

**Revendications**

1. Procédé de détermination des caractéristiques en vieillissement d'une fibre, dans lequel la fibre est courbée suivant un certain nombre de spires, dont le diamètre varie entre une valeur maximum et une valeur minimum, et est fixée dans cet état, caractérisé en ce que l'apparition d'une série de fractures dans la fibre, résultant de la charge statique, est enregistrée par un élément de détection, de même que l'instant d'apparition des fractures, lesquelles informations enregistrées par ledit élément de détection sont transférées vers un élément de traitement, qui calcule, sur la base desdites informations, le coefficient de corrosion sous contrainte, n, de la fibre en utilisant la formule suivante:

$$n = N/(\ln(R) \, \Delta k/\Delta \ln(ts))$$

où

N désigne le nombre de spires,
R désigne la relation entre le rayon de la spire maximum et celui de la spire minimum,
$\Delta k/\Delta \ln(ts)$ désigne la vitesse de variation des données de mesure, où k désigne le numéro d'ordre d'une fracture et ts l'instant d'apparition de la fracture correspondante, dans lequel le coefficient de corrosion sous contrainte, n, définit les caractéristiques en vieillissement de la fibre.

2. Procédé selon la revendication 1, caractérisé en ce que le diamètre des spires est déterminé par le diamètre et les caractéristiques de résistance de la fibre.

3. Dispositif, comprenant un élément présentant au moins une surface partiellement circulaire, dont le diamètre est déterminé par le diamètre et les caractéristiques de résistance d'une fibre, destiné à déterminer la durée de vie de la fibre, caractérisé en ce que la fibre est montée sur l'élément, sur la surface au moins partiellement circulaire, suivant un certain nombre de spires pendant une durée au cours de laquelle un élément de détection enregistre l'apparition d'une série de fractures dans la fibre et l'instant d'apparition de chaque fracture, les informations sur le nombre de ruptures et sur les instants de rupture étant transférées à partir dudit élément de détection vers un élément de traitement qui calcule le coefficient de corrosion sous contraintes, n, de la fibre, en utilisant la formule:

$$n = N/(\ln(R) \, \Delta k/\Delta \ln(ts)),$$

où

N désigne le nombre de spires,
R désigne la relation entre le rayon de la spire maximum et celui de la spire minimum,
$\Delta k/\Delta \ln(ts)$ désigne la vitesse de variation des données de mesure, où k désigne le numéro d'ordre d'une fracture et ts l'instant d'apparition de la fracture correspondante, dans lequel le coefficient de corrosion sous contraintes, n, définit les caractéristiques de vieillissement de la fibre, et en ce que les diamètres maximum et minimum de la surface partiellement circulaire sont déterminés par rapport au diamètre et aux caractéristiques de résistance connues de la fibre essayée.

4. Dispositif selon la revendication 3, caractérisé en ce que l'élément est conique.

5. Dispositif selon la revendication 3, caractérisé en ce que la fibre est montée sur l'élément suivant une ou plusieurs spires, la détection des fractures de la fibre étant réalisée, par exemple, de manière acoustique, mécanique ou optique en utilisant un élément de détection.

6. Dispositif selon la revendication 3, caractérisé en ce que l'élément est cylindrique et la fibre, après avoir été enroulée autour de l'élément, est soit relâchée, ou si le cylindre est réalisé en un matériau déformable, reste en place sur le cylindre, après quoi les différentes spires de la fibre, par une action mécanique, sont amenées à s'enrouler suivant différents diamètres, qui sont définis entre des valeurs maximum et minimum.

7. Dispositif selon les revendications 3 à 6, caractérisé en ce que le diamètre de courbure de la fibre autour de la surface circulaire indique la charge à laquelle la fibre est soumise.

Fig.1

Fig. 2

Fig.3

Fig. 4

Fig.5

A

B